# EUROPEAN PATENT SPECIFICATION

(11) **EP 1 401 464 B1**
(45) Date of publication and mention of the grant of the patent: **06.09.2006**
(21) Application number: 02749285.9
(22) Date of filing: 01.07.2002
(51) Int. Cl.: A61K 36/752, A61K 36/63, A61K 131/00, A61P 3/06

(54) **DIETETIC PREPARATION WITH HYPOCHOLESTEROLEMIC ACTIVITY**
DIÄTETISCHES MITTEL MIT HYPOCHOLESTEROLEMISCHER AKTIVITÄT
PREPARATION DIETETIQUE A ACTIVITE HYPOCHOLESTEROLEMIQUE

(30) Priority: 02.07.2001 IT MI20011404
(43) Date of publication of application: 31.03.2004
(73) Proprietor: Ansovini, Raffaele, 06126 Perugia (IT)
(72) Inventor: ANSOVINI RAFFAELE, I-06126 Perugia (IT)
(74) Representative: Long, Giorgio
(86) International application number: PCT/IT2002/000434
(87) International publication number: WO 2003/004040

(56) References cited:
- WO-A-99/32589
- DE-A- 3 900 447
- FR-A- 2 563 109
- DATABASE WPI Section Ch, Week 199633 Derwent Publications Ltd., London, GB; Class D13, AN 1996-323069 XP002229727 & ES 2 087 025 A (SORIA NATURAL SA), 1 July 1996 (1996-07-01)
- DATABASE WPI Section Ch, Week 199713 Derwent Publications Ltd., London, GB; Class B05, AN 1997-143802 XP002229728 & RU 2 063 766 C (HEALTH CENTRE), 20 July 1996 (1996-07-20)
- KOH D: "SPONTANEOUS PASSAGE OF GALLSTONES AFTER INGESTION OF OLIVE OIL A CASE REPORT" SINGAPORE MEDICAL JOURNAL, vol. 27, no. 6, 1986, pages 533-536, XP001106672 ISSN: 0037-5675

## Description

The present invention refers to a dietary preparation with hypocholesterolemic activity. In particular, the present invention refers to a lemon olive oil obtained from the combined pressing of olives and lemons such as to an ideal balance regarding the content of the active ingredients and therefore maximise activity for the reduction of the levels of cholesterol and other harmful fats in the blood.

In the field of nutrition, it has been scientifically proven of various food types typical of the Mediterranean area (olive oil, vegetables, citrus fruits, fish, etc.), known by the name of Mediterranean diet, is apt for the prevention of both cardiovascular diseases and the systemic damage caused by oxidising molecules, either endogenous or present in many foods. Both olives and citrus fruits contain active ingredients useful in the control of high levels of cholesterol and other fats in the blood and are therefore essential for the inhibition of the formation of atheromatous plaques which play a key role in numerous cardiovascular pathologies. Through continued dietary consumption, of the active ingredients contained in olive oil and lemons it is possible to obtain a safeguard for the circulatory system in general, and of the coronary circuit in particular.

In an era of dietary habits rich in fats such as ours, to have available the dietary factors which daily help to prevent these cardiovascular pathologies, the incidence of which is always more frequent in recent years, is a particularly felt need. At the same time, it is important that these dietary preparations have a pleasant taste, so as to be accepted by the consumer for daily and prolonged use.

ES 2087025 discloses a cholesterol-free sauce containing lemon juice and olive oil. No indication of a possible hypocholesterolemic activity is made indeed.

RU 2063766 discloses a two-stage treatment that includes administering herbal infusions, apple juice, vitamin infusions, fermented cow's milk and, among other ingredients, a mixture of lemon juice and olive oil. No specific disclosure of the use of an olive oil obtained by pressing together whole lemons and olives is made.

Koh D., Singapore Medical Journal, vol. 27, no. 6, 1986 reports of a case of remission of cholesterol gallstones after administration of olive oil and lemon juice.

FR-A-2563109 describes the use of a composition containing olive oil, essential lemon oil, chlorophyll and several essential oils in the treatment of respiratory diseases.

The inventor of the present patent application has surprisingly found that by conjointly pressing olives and lemons in determined weight ratios it is possible to obtain a dietary preparation in which the active ingredients with hypocholesterolemic and antioxidant activities are present in ideal and synergic ratios to allow an effective control of the cardiovascular pathologies in an individual following prolonged use.

The dietary preparation according to the present invention can be obtained through the combined pressing of olives and lemons in a weight ratio comprised of between 0.80 and 0.90 kg of lemons per kg of olives. Preferably, this weight ratio will be comprised of between 0.84 and 0.88 kg of lemons per kg of olives, more preferably it will be 0.86 kg of lemons per kg of olives.

It is important that the lemons have the skins included, in that, in the latter are contained many of the active ingredients useful in the cure of the listed pathologies. Preferably, the lemons will be cut prior to squeezing.

It is clearly essential that both the olives and the lemons have been cultivated according to organic culture dictates, that is they do not contain, not even in trace amounts, fungicidal compounds.

The product of the pressing will not be refined according to the procedures normally employed for oils and as such will be in the form of an extra virgin, lemon olive oil.

The pressing will happen preferably in the cold and will be soft.

The methods used for pressing are these normally used by experts in the sector for the pressing of olives and will therefore not be described in further detail.

The dietary preparation according to the present invention can be administered as it is or as a dressing, as part of a normal diet. Preferably, it must be consumed raw.

The advised usage is daily, even if in certain cases administration on alternate days or, at least, two or three times a week could be sufficient.

The amount of dietary preparation preferred for daily use is that normally used for raw oil as a dressing, or two or three spoonfuls per day.

It is also envisaged that the lemon-oil according to the present invention, be used in association with pharmaceutically acceptable excipients- and in a pharmaceutically efficacious dose, in the preparation of a medicament - with hypocholesterolemic, antiatheromatous and antioxidant activities. A pharmaceutical formulation thus obtained also constitutes a subject of the present invention.

The advantages of the lemon-oil according to the present invention are both to have obtained a perfect balance and a synergic effect between the active ingredients present in the starting products (olives and lemons), and to have made available a dietary preparation with a particularly pleasant taste, so as to induce the consumer towards its daily use.

## Claims

1. Dietetic preparation obtainable from the combined pressing of olives and lemons in a ratio comprised of between 0.08 and 0.90 kg of lemons per kg of olives, in which the lemons are pressed with the skins included.

2. Dietetic preparation according to claim 1, in which the lemons are present in a weight ratio comprised of between 0.84 and 0.88 kg per kg of olives, preferably 0.86 kg per kg of olives.

3. Dietetic preparation according to any one of the claims from 1 to 2, in which the lemons have been previously out.

4. Dietetic preparation according to any one of the claims from 1 to 3, in which said pressing is a soft and cold pressing.

5. Use of the dietetic preparation according to any one of the claims from 1 to 4 for the preparation of a medicament hating hypocholesterolemic, antiatheromatous and antioxidant activities.

6. Pharmaceutical formulation comprising a pharmaceutically efficacious dose of the dietetic preparation according to any one of the claims from 1 to 4 in association with pharmaceutically acceptable excipients.

## Patentansprüche

1. Diätetische Zubereitung, erhältlich durch gemeinsame Pressung von Oliven und Zitronen in einem Verhältnis von 0,80 bis 0,90 kg Zitronen pro kg Oliven, wobei die Zitronen mit Schalen gepresst werden.

2. Diätetische Zubereitung nach Anspruch 1, worin die Zitronen in einem Gewichtsverhältnis von 0,84 bis 0,88 kg pro kg Oliven, und vorzugsweise 0,86 kg pro kg Oliven vorliegen.

3. Diätetische Zubereitung nach einem der Ansprüche 1 bis 2, bei der die Zitronen vorher geschält wurden.

4. Diätetische Zubereitung nach einem der Ansprüche 1 bis 3, bei der die Pressung eine sanfte und kalte Pressung ist.

5. Verwendung der diätetischen Zubereitung nach einem der Ansprüche 1 bis 4 zur Herstellung eines Medikaments mit hypocholesterinämischen, antiatheromatösen und antioxidativen Wirkungen.

6. Pharmazeutische Zusammensetzung, umfassend eine pharmazeutisch wirksame Dosis der diätetischen Zubereitung nach einem der Ansprüche 1 bis 4 zusammen mit pharmazeutisch geeigneten Trägern.

## Revendications

1. Préparation diététique susceptible d'être obtenue à partir de la pression combinée d'olives et de citrons dans un rapport compris entre 0,80 et 0,90 kg de citrons par kg d'olives, dans laquelle les citrons sont pressés avec les peaux comprises.

2. Préparation diététique selon la revendication 1, dans laquelle les citrons sont présents dans un rapport en poids compris entre 0,84 et 0,88 kg par kg d'olives, de préférence 0,86 kg par kg d'olives.

3. Préparation diététique selon l'une quelconque des revendications 1 à 2, dans laquelle les citrons ont été préalablement coupés.

4. Préparation diététique selon l'une quelconque des revendications 1 à 3, dans laquelle ladite pression est une pression douce et à froid.

5. Utilisation de la préparation diététique selon l'une quelconque des revendications 1 à 4 pour la préparation d'un médicament ayant des activités hypocholestérolémiques, antiathéromateuses et antioxydantes.

6. Formulation pharmaceutique comprenant une dose pharmaceutiquement efficace de la préparation diététique selon l'une quelconque des revendications 1 à 4 en association avec des excipients pharmaceutiquement acceptables.
